# EUROPEAN PATENT SPECIFICATION

(11) **EP 1 738 790 B1**
(45) Date of publication and mention of the grant of the patent: **17.10.2007**
(21) Application number: 05013831.2
(22) Date of filing: 27.06.2005
(51) Int. Cl.: A61M 25/00, A61L 29/00

(54) **Device for the injection of drugs into microvessels**
Vorrichtung zur Injektion von Medikamenten in Mikrogefässe
Dispositif pour l'injection de médicaments dans des microvaisseaux

(43) Date of publication of application: 03.01.2007
(73) Proprietor: Hattenbach, Lars-Olof, 65835 Liederbach (DE); Hilgenberg, Ingo, 34323 Malsfeld (DE); Mann, Dieter, 63814 Mainaschaff (DE)
(72) Inventor: Hattenbach, Lars-Olof, 65835 Liederbach (DE); Hilgenberg, Ingo, 34323 Malsfeld (DE)
(74) Representative: Hofer, Dorothea

(56) References cited:
- DE-A1- 3 218 167
- DE-C1- 3 512 018
- US-A- 5 817 075
- US-A1- 2002 133 101
- US-A1- 2003 057 347
- US-B1- 6 224 566
- US-B1- 6 402 734

## Description

The invention relates to a device for the injection of drugs into microvessels, in particular into retinal microvessels.

Retinal vessel occlusion (RVO) is the most common form of retinal vascular disease after diabetic retinopathy. The natural course of central retinal vein or artery occlusion is associated with a poor visual prognosis and a high incidence of complications. Further, retinal vessel occlusion is among the most common retinal diseases in the Western World. In view of an increasing number of elderly people with cardiovascular diseases, the development of therapeutic approaches to the management of retinal vessel occlusion is a major medical and socio-economic goal.

In retinal artery occlusion, the most common symptom is an acute, painless loss of vision in one eye. The degree of loss depends on the location of the occlusion. If the occlusion occurs in the central artery of the retina (CRAO), damage usually results in permanent complete loss of vision in the affected eye. If occlusion occurs in a branch artery (BRAO), vision loss will be partial. On ophthalmoscopy, central retinal artery occlusion is characterized by a pale, whitish-yellow ischemic retina and a cherry-red spot in the center of the macula. The blockage of blood flow to the retina is typically caused by an embolus (clot) in the blood stream. The occlusion decreases the oxygen supply to the area of the retina nourished by the affected artery.

In retinal vein occlusion, typical findings on ophthalmoscopy are retinal hemorrhages, marked retinal edema and cotton wool spots, disc swelling and tortuous and dilated retinal veins. If there is a central retinal vein occlusion (CRVO), these findings will encompass all four retinal quadrants. A hemi-central retinal vein occlusion will involve only the superior or inferior half of the retina. A branch retinal vein occlusion (BRVO) will present with findings in only one quadrant, usually supero-temporal, with the apex of the hemorrhage at an arteriovenous crossing. Additionally, retinal ischemia induces the formation of pathologic blood vessels (neovascularization) in the anterior segment of the eye, which leads to neovascular glaucoma. This has been reported in 16 % to 67 % of all eyes with CRVO, usually within 90 days after the initial examination (Hayreh SS, Rojas P, Podhajsky P, Montague P, Woolson RF. Ocular neovascularization with retinal vascular occlusion-III. Incidence of neovascularization with retinal vein occlusion. Ophthalmology 1983;90:488-506). Left untreated, as many as 93 % of severe CRVO may culminate in a final visual acuity of less than or equal to 20/200 (Quinlan PM, Elman MJ, Bhatt AK, Mardesich P, Enger C. The natural course of central retinal vein occlusion. Am J Ophthalmol 1990;110:118-123).
The morphological features of retinal vein occlusion include endothelial cell and intimal proliferation, chronic inflammation, phlebosclerosis, and thrombus formation (Green WR, Chan CC, Hutchins GM, Terry JM. Central retinal vein occlusion: A prospective histopathologic study of 29 eyes in 28 cases. Retina 1981;1:27-55; Smith P, Green WR, Miller NR, Terry JM: Clinicopathologic case report of bilateral central retinal vein occlusion in Reye's syndrome. Arch Ophthalmol 1980;98:1256-1260).

To date, the mainstay of therapy is by panretinal photocoagulation (laser treatment) to prevent or treat neovascularization. It is, however, not effective in restoring sight that has been lost prior to treatment. Current therapeutic approaches include hemodilution, the systemic administration of anticoagulant or antiplatelet drugs or various non-endovascular surgical approaches such as adventitial sheathotomy in branch retinal vein occlusion or radial optic neurotomy (RON) in central retinal vein occlusion.

To date, there is still no consensus among ophthalmologists about the management of retinal vein or artery occlusion.

A reduction in retinal ischemia can be achieved by laser therapy (photocoagulation), which results in significant suppression of neovascularization. However, laser treatment is not effective in restoring sight that has been lost prior to treatment.

Hemodilution to reduce the viscosity of the blood and thereby increase the perfusion of the retina is among the medical treatment options in both, retinal vein or retinal artery occlusion. However, although several studies provide evidence that hemodilution has a beneficial effect in retinal vein occlusion. The true value of this therapeutic approach in retinal artery occlusion or severe cases of CRVO remains questionable.

Several authors advocate the oral administration of hemorheologic agents whereas others support consideration of non-endovascular surgical approaches including adventitial sheathotomy (arteriovenous dissection) in BRVO. However, currently, no medical treatment exists that has been shown to definitively improve vision in RVO.

The finding that thrombus formation is a prominent feature of retinal vascular occlusive disease supports consideration of thrombolysis aimed at early restoration of blood flow. During the past decade, thrombolytic therapy has become the standard care for patients with acute myocardial infarction. Moreover, the spectrum of indications for thrombolytic drugs such as streptokinase or recombinant tissue plasminogen activator (rt-PA) comprises lung embolism, ischemic stroke, deep vein thrombosis and acute arterial occlusions of the lower limbs. Several studies suggest that thrombolysis with rt-PA may be associated with a better visual prognosis in retinal vein or artery occlusion.

Furthermore, the consideration of thrombolytic therapy in ischemic CRVO is supported by the morphological features of retinal vein occlusion which include thrombus formation. In accordance with the histopathological features, various thrombophilic abnormalities have been found to be associated with retinal vein occlusion. The risk of bleeding is the major problem with fibrinolysis. Thus, the choice of an appropriate thrombolytic therapy in a non-life threatening situation such as retinal vessel occlusion should be based on minimizing the probability of potential complications such as cerebral or gastrointestinal hemorrhage. Furthermore, the fibrinolytic treatment of choice should be able to produce rapid and complete restoration of retinal capillary and venous blood flow. In light of the fact that the occurrence of hemorrhagic complications constitutes a dose dependent problem, the puncture of retinal vessels with targeted delivery of fibrinolytic agents at low doses appears to be the ideal approach.

Over the past years, various methods for the puncture of retinal vessels have been proposed (Cunha-Vaz JG, Murta JN, Proenca RD. Micropuncture of retinal vessels. Dev Ophthalmol 1989;18:90-4; Glucksberg MR, Dunn R, Giebs CP. In vivo micropuncture of retinal vessels. Graefes Arch Clin Exp Ophthalmol 1993;231:405-7).

Recent approaches include the cannulation of retinal veins with micropipettes (Weiss JN. Treatment of central retinal vein occlusion by injection of tissue plasminogen activator into a retinal vein. Am J Ophthalmol 1998;126:142-4; Weiss JN, Bynoe LA. Injection of tissue plasminogen activator into a branch retinal vein in eyes with central retinal vein occlusion. Ophthalmology 2001; 108:2249-2257; Suzuki J, Matsuhashi H, Nakazawa M. In vivo retinal vascular cannulation in rabbits. Graefes Arch Clin Exp Ophthalmol 2003;241:585-8) or the catheterization of retinal vessels using a flexible microcatheter (Tameesh MK, Lakhanpal RR, Fujii GY, Javaheri M, Shelley TH, D'Anna S, Barnes AC, Margalit E, Farah M, De Juan E Jr, Humayun MS. Retinal vein cannulation with prolonged infusion of tissue plasminogen activator (t-PA) for the treatment of experimental retinal vein occlusion in dogs. Am J Ophthalmol 2004;138:829-39) for the injection of the fibrinolytic agent rt-PA (recombinant tissue-plasminogen activator).

However, although these methods have demonstrated the feasibility of retinal vessel puncture, there is still a lack of clinically applicable methods and devices for the injection of drugs into the lumen of retinal vessels in a reliable manner. As a result, endovascular surgery of retinal vessels is still far from becoming a routine procedure in the management of retinal vascular occlusive diseases.

The major problem with available techniques is the limited visualization of the retinal vessel due to an insufficient magnification with conventional surgical microscopes, making it difficult to puncture the vessel or effectively control the targeted injection of drugs.

In previous attempts to facilitate the task of delivering drugs to retinal microvessels, various complex micromanipulation devices or expensive robots have been used (Weiss JN, Bynoe LA. Injection of tissue plasminogen activator into a branch retinal vein in eyes with central retinal vein occlusion. Ophthalmology 2001;108:2249-2257; Jensen PS, Grace KW, Attariwala R, Colgate JE, Glucksberg MR. Toward robot-assisted vascular microsurgery in the retina. Graefes Arch Clin Exp Ophthalmol 1997;235:696-701). Such micromanipulators require a maximum stability, which is usually accomplished by a complex fixation of the patient's head and/or eye, thereby limiting accessibility and operability in a surgical setting. Moreover, micromanipulation devices do not overcome the problem of visualization.

With the advent of high resolution gradient index (GRIN) microendoscopes, the implementation of a safe and reliable microsurgical technique for the puncture of retinal vessels has become technically feasible. However, although the endoscopic cannulation of retinal veins has been attempted earlier, this approach remained in its infancy. In former experimental investigations, a GRIN endoscope was used to visualize the introduction of a simple handheld glass micropipette (Hamza HS, Humayun MS, Jensen PS, Shelly T, Shoukas A, de Juan Jr E. Endoscopic guided hand-held cannulation of the retinal veins in vivo. Invest Ophthalmol Vis Sci 1999;40(Suppl):768).

Another major concern is the size and form of current devices for the puncture of retinal microvessels. Thus far, cannulas or microcatheters have been designed to be insertable into main branches close to the optic nerve head. Usually, these vessels are at least 100 µm in diameter. Since the maximum achievable reduction in size of a cannula depends on stability, previous techniques using materials such as "standard" glass (e.g. borosilicate), metal or polyamide have been limited to an outer diameter ranging from 100 to a minimum of 50 µm.

Moreover, the tip design of cannulas used in previous approaches mainly has been adapted to the shape of standard size cannulas used for the puncture of major vessels. However, the ideal design for the puncture of microvessels should aim at minimizing the damage to the vessel wall.

US 6,402,734 B1(closest prior art) and US 2003/0057347 A1 disclose a micropipette/microcannula for cannulating a retinal blood vessel. The microcannula is preferably made of glass and consists of an elongated non-bendable, relatively rigid hollow body member having a single angled first end to define a relatively sharp bevelled tip and a second end which is attached to a flexible tubing member. The bevelled tip end has an outer diameter of approximately 100 µm and an inner diameter of approximately 72 µm.

WO 01/49352 A2 discloses a microcatheter system that allows for a vascular infusion into retinal veins for extended periods of time. The microcatheter system includes a flexible cannula that is inserted into the retinal vein lumen and that remains stably within the retinal vein lumen without being held by a robot, micromanipulator or similar holding devices. The cannula is fabricated of polyimide or similar material. The outer diameter is from about 50 µm to about 80 µm. The cannula has a ramp-like distal end which has an angle of about 30°.

The stability is an important prerequisite for the safe routine use of microinjection devices. The materials used in the previous attempts to cannulate retinal vessels do not perfectly meet these requirements. The size and the shape of the cannula are crucial for the usefulness of microinjection techniques. For example, the management of branch retinal vein occlusion would require the injection of a fibrinolytic agent proximally to the occluded portion of the vein, i.e. the puncture of a vessel as small as 50 µm or less in diameter. Moreover, the puncture of retinal vessels in close proximity to the optic disk carries the risk of damaging the optic nerve.

It is an object of this invention to provide a device that can be used in a standard vitreoretinal surgery setting in combination with a standard ophthalmic surgery endoscope in order to perform a safe and controlled puncture of retinal vessels with or without the injection of drugs under optimal visualization, without the support of complex micromanipulation devices. Furthermore, it is an object of the present invention to provide a cannula that is resistant to mechanical damage, though small enough to be insertable into the lumen of microvessels with a smaller diameter such as branch retinal veins or arteries.

The object is solved by a device according to claim 1 or claim 14 or claim 15. Further developments are given in the dependent claims.

The invention provides for a device for the endoscopically guided puncture of retinal or other microvessels which is particularly effective in the treatment of retinal vascular occlusive diseases.

The microcatheter and the microcatheter system of the present invention have in particular the following advantages. The microcatheter has an extremely small sharp tip which is, however, large enough to allow for the injection of fluid. The material of the microcatheter is robust enough to sustain the process of sharpening the tip. Moreover, it provides for a mechanically stable catheter which is a prerequisite for the safe routine use of microinjection devices.

The system is designed for the injection of fluid pharmacologic substances such as fibrinolytic agents in the management of retinal vessel occlusion. It may be used in a standard vitreoretinal surgery setting, without the support of robots, microdrives or other micromanipulators.

The catheter is fabricated of a robust, though flexible material, which is a quartz glass tubing, preferably coated with a polyamide. The catheter is made of a single piece which has an advantage over assembled systems with glued or welded components, since leakage secondary to the high intraluminal pressures required for the injection of fluids through tubings with extremely small diameters may be prevented. The total length of the single piece microcatheter may be variable, extending up to 100 cm or more. Quarz glass conists of pure SiO₂ and does not have additives such as sodium carbonate, calcium carbonate or boron, which are present in the glass type used for the known catheters. Quarz glass has the advantage of having a high mechanical stability, a small thermal expansion and high transmittance for ultra violet light, the latter being of advantage for the sterilization of the material.

Preferably, the catheter is attached along a tube ("sleeve") made of glass, metal or a synthetic polymer. The size of this tube may be adapted to a particular standard endoscope used in vitreoretinal surgery setting, with an inner diameter ranging from approximately 0.5 to 1.2 mm and a length ranging from approximately 20 to 30 mm.

Preferably, the sleeve carrying the catheter is mounted on the fiberoptic tip of the endoscope, creating a single instrument. This has an advantage over settings with multiple instruments, since the bimanual manipulation of a single device allows for precise manoeuvres by the surgeon without the support of complex micromanipulators.

Once mounted on the endoscope, the sharp end of the catheter is positioned in front of the tip of the endoscope, allowing for the simultaneous visualisation of the catheter and the target vessel.

Preferably, the sharp end of the catheter is coated, e.g. with a gold layer to enhance visualisation. In addition, standard dyes such as indocanine green (ICG) may be used as an adjunct to injected pharmacologic substances.

To allow for an optimal visualisation and positioning parallel to the target vessel, the sharp end of the catheter is angled, approximately between 40° to 60°.

In contrast to other simple bevelled ramp-like tip designs, the end of the catheter is tapered in order to maximally reduce thickness of the tip. As a result, the tip of the catheter has an extremely small outer diameter ranging from 50 µm to less than 10 µm up to 5 µm. The diameter of the tip of the sharp end may be variable, depending on the size of targeted microvessel. In addition, the tip of the tapered catheter end is sharpened and may be equipped with an additional spike to increase sharpness.

The opposite end of the catheter is equipped with a Luer Lock adaptor, resistant to high intraluminal pressures, for the connection of an infusion line. Preferably, the infusion line is supplied by an automated syringe or high pressure infusion system, controlled by the surgeon, e.g. with the help of a foot pedal.

In a standard situation, the surgical removal of vitreous body (pars plana vitrectomy) may be performed prior to the puncture of the microvessel. The sleeve of the catheter system is then mounted on the tip of the endoscope, so that the sharp end of the catheter is positioned in front of the tip of the endoscope and introduced manually into the vitreous cavity through a scleral incision (sclerotomy) at a distance of 3-3.75 mm from the limbus.

In order to protect the sharp tip of the catheter, the introduction of the system through the sclerotomy is performed with a spreader or other protection device, e.g. a removable cover. The combined endoscope-catheter system may then be moved freely within the vitreous cavity, manually controlled by the surgeon. Visualisation of the vitreous cavity and the retinal target vessel are provided simultaneously by the surgical standard microscope and the endoscopic view, displayed on a monitor.

By moving the endoscope, the sharp end of the catheter is positioned parallel to the retinal target vessel. Cannulation and injection of fluids or drugs are then performed under direct visualisation with the endoscope. Because of the extremely high magnification provided by the endoscopic view, cannulation and injection can be performed in a safe, reliable and reproducible manner. The catheter may then be removed immediately or remain within the microvessel for a period of time required for a specific treatment, as desired by the surgeon.

Further features and advantages will become apparent from the description of an embodiment of the invention in conjunction with the accompanying figures.
Fig. 1 shows a schematic view of one embodiment of the catheter system of the invention.
Fig. 2a shows an enlarged view of the end portion of the catheter mounted to the tip of an endoscope.
Fig. 2b shows an enlarged portion of Fig. 2a.
Fig. 3 shows a schematic view of the surgical approach.
Fig. 4 shows a principle of a surgical procedure according to the invention.

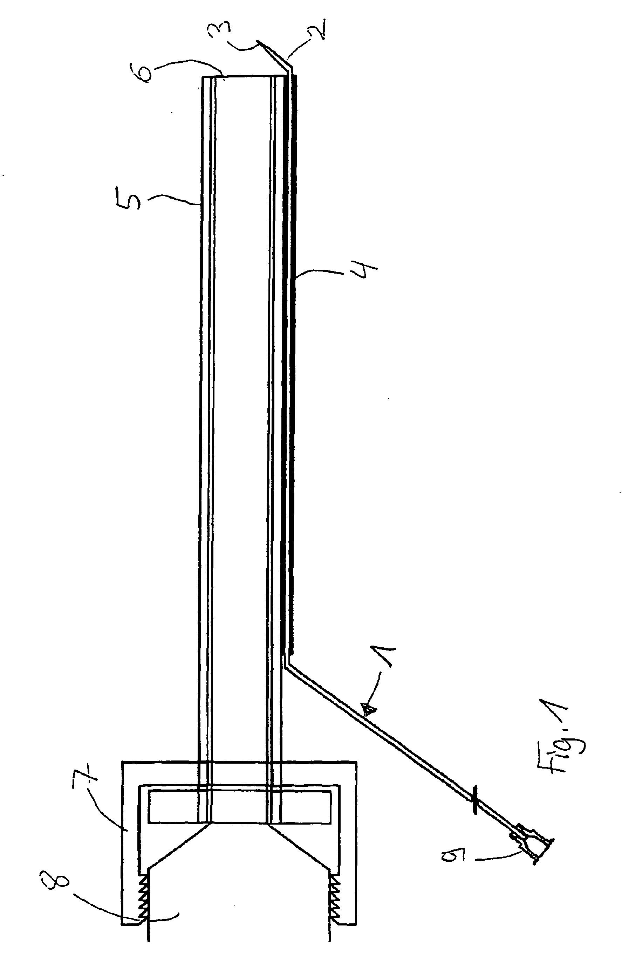

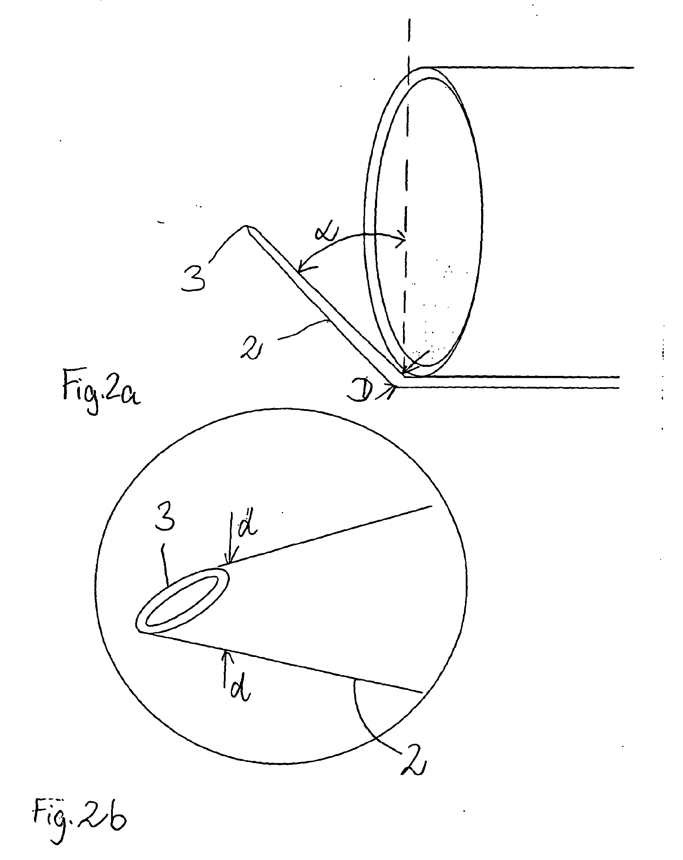

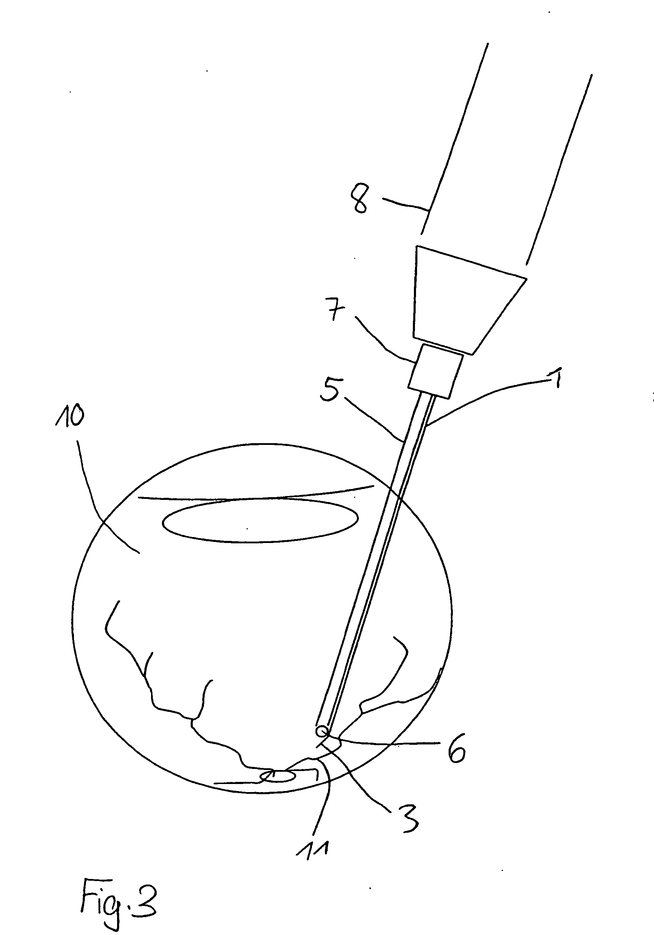

An embodiment of the catheter and the catheter system of the invention is now described with reference to Figs. 1 and 2a, b. As can be seen from Fig. 1 the catheter 1 is made of a single piece tubing with a total length up to approximately 100 cm or even more. The outer diameter in this embodiment is approximately 100 µm and the catheter has a thin wall of about 20 µm to 30 µm in the flexible part. The catheter 1 is fabricated from a quartz glass. Because of its relatively small diameter and relatively large total length the catheter is flexible. At one end the catheter 1 comprises an end portion 2 with a sharp tip 3. The main part of the flexible catheter 1 is attached, for example, by means of a guiding tube 4 to a tube or sleeve 5 made of glass, metal or a synthetic polymer which is mounted on the fiberoptic tip 6 of a standard endoscope used in a vitreoretinal surgery setting. The inner diameter of the sleeve 5 is in the range of approximately 0.5 to 1.2 mm. The length of the sleeve is in the range of approximately 20 to 30 mm. The sleeve 5 is mounted with a nut 7 on the handle part 8 of the endoscope. In this way a single instrument is created which can be held and controlled in one hand by the surgeon.

As can be seen in particular in Figs. 1 and 2a the end portion 2 is bent in front of the free end of the sleeve 5 at an angle α of approximately 40° to approximately 60°. Once mounted on the endoscope, the sharp tip 3 of the catheter 1 is positioned in front of the tip 6 of the endoscope, allowing for the simultaneous visualisation of the catheter and the target vessel. The length of the end portion 2 is such that the end portion 2 is no longer flexible, but rigid to allow for a precise incision in the target vessel. As can be seen in particular in Fig. 2 the end portion 2 tapers towards the sharp tip 3 in a conical fashion. The diameter D at the transition of the main body of the catheter and the end portion 2 decreases to a diameter d at the sharp tip 3 ranging from about 50 µm to less than 10 µm, more specifically about 30 µm to 5 µm. In addition, the sharp tip 3 is bevelled as can be seen from Fig. 2b. Hence, the sharp tip 3 of the catheter is finely drawn-out so as to maximally reduce the thickness of the tip in contrast to other simple bevelled ramp-like tip designs.

Dependent on the size of the targeted microvessel the sharp tip 3 may be fabricated with another diameter. In addition, the tip may be sharpened and/or equipped with an additional spike to increase sharpness.

The whole end portion 2 and/or the sharp tip 3 may be coated or plated, e.g. with a gold layer to enhance visualisation. In addition, standard dyes such as indocanine green (ICG) may be used as an adjunct to injected pharmacologic substances.
The whole catheter 1 can be coated, for example with a polyamide coating. The stability and/or flexibility may be enhanced with the coating.

At its other end, opposite to the sharp tip, the catheter is equipped with a Luer Lock adapter 9 for the connection of an infusion line (not shown). The Luer Lock adapter is resistant to high intraluminal pressures. Preferably, the infusion line is supplied by an automated syringe or high pressure infusion system, controlled by the surgeon, e.g. with the help of a foot pedal.

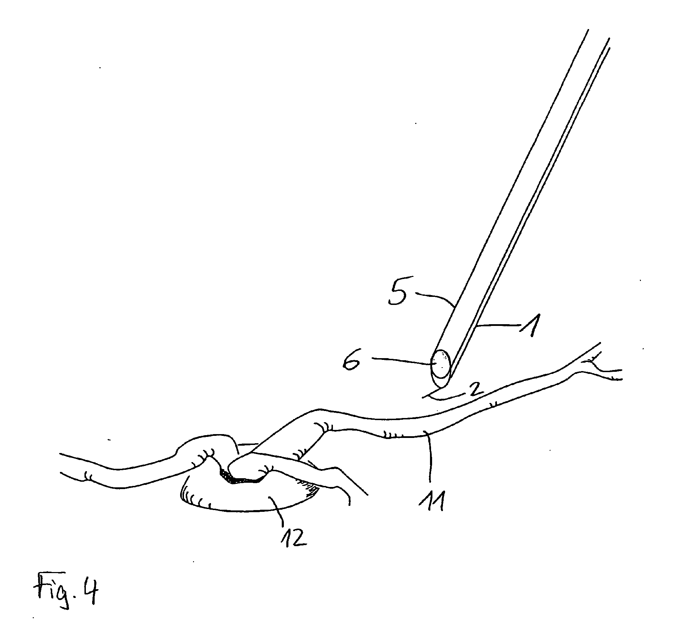

The application of the microcatheter system will now be described with reference to Figs. 3 and 4. First, the sleeve 5 of the catheter system is mounted on the tip 6 of the endoscope, so that the sharp tip 3 of the catheter 1 is positioned in front of the tip 6 of the endoscope and introduced manually into the vitreous cavity 10 through a scleral incision (sclerotomy) at a distance of about 3-3.75 mm from the limbus. Dependent on the size of the targeted microvessel catheter 1 with a sharp tip 3 with a required diameter is selected in advance. Fig. 3 shows the sleeve 5 carrying the single piece catheter 1 mounted on the fiberoptic tip 6 of an endoscope with the nut 7 which connects the catheter system to the handle 8 of the endoscope. The sleeve 5 is placed within the vitreous cavity 10. In Fig. 4 reference numeral 12 indicates the optic disc. The sharp tip 3 of the catheter is positioned parallel to a target vessel 11. As shown in Fig. 4 the fiberoptic tip 6 of the endoscope with the mounted sleeve 5 carrying the catheter 1 is positioned over the retinal target vessel 11. The sharp tip 3 of the catheter 1 is then placed parallel to the vessel 11 and introduced into the lumen under direct visualisation and control with the endoscope. Cannulation and injection of fluids or drugs are then performed under direct visualisation with the endoscope. Because of the extremely high magnification provided by the endoscopic view, cannulation and injection can be performed in a safe, reliable and reproducible manner. The catheter 1 may then be removed immediately or remain within the microvessel for a period of time required for a specific treatment, as desired by the surgeon.

In a standard situation, the surgical removal of the vitreous body (pars plana vitrectomy) may be performed prior to the puncture of the microvessel.

In order to protect the sharp tip 3 of the catheter 1 the introduction of the system through the sclerotomy is performed with a spreader or another protection device, e.g. a removable cover. The combined endoscope-catheter system may then be moved freely within the vitreous cavity, manually controlled by the surgeon. Visualisation of the vitreous cavity and the retinal target vessel are provided simultaneously by the surgical standard microscope and the endoscopic view, displayed on a monitor.

The invention is not limited to the embodiment described above. The length and diameter of the main body of the catheter may vary dependent on the application, for example, dependent on the endoscope used. The sharp tip 3 can be hardened. The material of the catheter should be a glass which does not have additions of sodium carbonate, calcium carbonate or boron. The material may be hard glass.

The invention is further not limited to the use of the catheter together with an endoscope. The catheter may be used in connection with another surgical or diagnostic instrument, such as for example an aspiration needle, and can also be mounted to a handle of such a surgical or diagnostic device.

The invention is further not limited to the application to retinal microvessels, but can be used for the injection of drug into other kind of microvessels.

The catheter may also be used for the surgery of vessels.

## Claims

1. A catheter for the injection of drugs in a microvessel, in particular in a retinal microvessel, the catheter comprising a single piece tubing (1), the tubing having an end portion (2) comprising a sharp tip (3) **characterised in that** the tubing is made of glass, which does not have additions of sodium carbonate, calcium carbonate or boron.

2. The catheter of claim 1, wherein said tubing is flexible.

3. The catheter of claim 1 or 2, wherein said end portion (2) is substantially rigid.

4. The catheter of one of claims 1 to 3, wherein said end portion (2) tapers towards said sharp tip (3).

5. The catheter of one of claims 1 to 4, wherein the outer diameter of said sharp tip (3) is in the range of approximately 30 µm to approximately 5 µm.

6. The catheter of one of claims 1 to 5, wherein said end portion (2) and/or said sharp tip (3) is coated to enhance the visibility.

7. The catheter of one of claims 1 to 6, wherein said sharp tip (-3) is provided with an additional spike.

8. The catheter of one of claims 1 to 7, wherein said sharp tip (3) is bevelled.

9. The catheter of one of claims 1 to 8, wherein the end opposite to the sharp tip (3) of the catheter (1) is provided with an adapter (9) for the connection with an infusion line.

10. A catheter system for the injection of drugs in a microvessel, in particular in a retinal microvessel, the system comprising
a catheter according to one of claims 1 to 9, and
a handle to which said catheter is mounted.

11. The catheter system of claim 10, wherein said handle is the handle of an endoscope.

12. The system of claim 11, wherein said catheter (1) is attached to a sleeve (5) which is mounted to the tip (6) of the endoscope.

13. The system of claim 11 or 12, wherein said end portion (2) extends at an angle relative to the tip of the endoscope, preferably at an angle between approximately 40° to 60°.

14. A catheter system for the injection of drugs in a microvessel, in particular in a retinal microvessel, the system comprising
a catheter (1) according to one of claims 1 to 9 comprising a tubing (1) and an end portion (2) with a sharp tip (3);
an endoscope having a fiberoptic tip (6) ,
wherein said catheter is attached to a sleeve (5) and said sleeve (5) is mounted on said fiberoptic tip (6).

## Patentansprüche

1. Katheter für die Injektion von Medikamenten in ein Mikrogefäß insbesondere ein Netzhaut-Mikrogefäß, wobei der Katheter eine einteilige Röhre (1) aufweist, welche einen Endabschnitt (2) mit einer scharfen Spitze (3) aufweist, **dadurch gekennzeichnet, dass** die Röhre aus Glas besteht, welches keine Zusätze an Natriumcarbonat, Kalziumcarbonat oder Bor aufweist.

2. Katheter nach Anspruch 1, wobei die Röhre flexibel ist.

3. Katheter nach Anspruch 1 oder 2, wobei der Endabschnitt (2) im Wesentlichen starr ist.

4. Katheter nach einem der Ansprüche 1 bis 3, bei dem der Endabschnitt (2) sich zu der scharfen Spitze (3) hin verjüngt.

5. Katheter nach einem der Ansprüche 1 bis 4, bei dem der Außendurchmesser der scharfen Spitze (3) in dem Bereich von annähernd 30 µm bis annähernd 5 µm ist.

6. Katheter nach einem der Ansprüche 1 bis 5, bei dem der Endabschnitt (2) und/oder die scharfe Spitze (3) beschichtet ist zum Erhöhen der Sichtbarkeit.

7. Katheter nach einem der Ansprüche 1 bis 6, bei dem die scharfe Spitze mit einem zusätzlichen Dorn versehen ist.

8. Katheter nach einem der Ansprüche 1 bis 7, bei dem die scharfe Spitze (3) abgeschrägt ist.

9. Katheter nach einem der Ansprüche 1 bis 8, bei dem das Ende gegenüber der scharfen Spitze (3) des Katheters (1) mit einem Adapter (9) für die Verbindung mit einer Infusionsleitung versehen ist.

10. Kathetersystem für die Injektion von Medikamenten in ein Mikrogefäß, insbesondere in ein Netzhaut-Mikrogefäß, wobei das System aufweist
einen Katheter nach einem der Ansprüche 1 bis 9 und
einen Griff, an den der Katheter montiert ist.

11. Kathetersystem nach Anspruch 10, bei dem der Griff der Griff eines Endoskops ist.

12. System nach Anspruch 11, bei dem der Katheter (1) an einer Hülle (5) angebracht ist, welche an der Spitze (6) des Endoskops montiert ist.

13. System nach Anspruch 11 oder 12, bei dem der Endabschnitt (2) sich unter einem Winkel relativ zu der Spitze des Endoskops erstreckt, vorzugsweise unter einem Winkel zwischen annähernd 40° bis 60°.

14. Kathetersystem für die Injektion von Medikamenten in ein Mikrogefäß, insbesondere in ein Netzhaut-Mikrogefäß, wobei das System aufweist
einen Katheter (1) nach einem der Ansprüche 1 bis 9, der eine Röhre (1) und einen Endabschnitt (2) mit einer scharfen Spitze (3) aufweist;
ein Endoskop mit einer Faseroptikspitze (6),
wobei der Katheter an einer Hülle (5) angebracht ist, und die Hülle (5) auf die Faseroptikspitze (6) montiert ist.

## Revendications

1. Cathéter destiné à l'injection de médicaments dans un microvaisseau, en particulier dans un microvaisseau rétinien, le cathéter comprenant
une tubulure en une seule pièce (1), la tubulure ayant une partie d'extrémité (2) comprenant un pointe affûtée (3), **caractérisée en ce que** la tubulure est formée par du verre auquel pas de carbonate de sodium, de carbonate de calcium ou de bore n'a été ajouté.

2. Cathéter selon la revendication 1, dans lequel ladite tubulure est flexible.

3. Cathéter selon la revendication 1 ou 2, dans lequel ladite partie d'extrémité (2) est substantiellement rigide.

4. Cathéter selon l'une des revendications 1 à 3, dans lequel ladite partie d'extrémité (2) se rétrécit vers ladite pointe affûtée (3).

5. Cathéter selon l'une des revendications 1 à 4, dans lequel le diamètre extérieur de ladite pointe affûtée (3) se trouve dans la plage d'approximativement 30 µm à approximativement 5 µm.

6. Cathéter selon l'une des revendications 1 à 5, dans lequel ladite partie d'extrémité (2) et/ou ladite pointe affûtée (3) est revêtue pour améliorer la visibilité.

7. Cathéter selon l'une des revendications 1 à 6, dans lequel ladite pointe affûtée (3) est dotée d'une pointe supplémentaire.

8. Cathéter selon l'une des revendications 1 à 7, dans lequel ladite pointe affûtée (3) est biseautée.

9. Cathéter selon l'une des revendications 1 à 8, dans lequel l'extrémité opposée à la pointe affûtée (3) du cathéter (1) est dotée d'un adaptateur (9) pour la connexion à une ligne de perfusion.

10. Système de cathéter destiné à l'injection de médicaments dans un microvaisseau, en particulier dans un microvaisseau rétinien, le système comprenant
un cathéter selon l'une des revendications 1 à 9, et
une poignée à laquelle ledit cathéter est monté.

11. Système de cathéter selon la revendication 10, dans lequel ladite poignée est la poignée d'un endoscope.

12. Système selon la revendication 11, dans lequel ledit cathéter (1) est fixé à un manchon (5) qui est monté à la pointe (6) de l'endoscope.

13. Système selon la revendication 11 ou 12, dans lequel ladite partie d'extrémité (2) s'étend en formant un angle par rapport à la pointe de l'endoscope, de préférence en formant un angle compris entre approximativement 40° et 60°.

14. Système de cathéter destiné à l'injection de médicaments dans un microvaisseau, en particulier dans un microvaisseau rétinien, le système comprenant
un cathéter (1) selon l'une des revendications 1 à 9 comprenant une tubulure (1) et une partie d'extrémité (2) avec une pointe affûtée (3) ;
un endoscope ayant une pointe (6) en fibre optique,
dans lequel ledit cathéter est attaché à un manchon (5) et ledit manchon (5) est monté sur ladite pointe (6) en fibre optique.
